# EUROPEAN PATENT SPECIFICATION

(11) **EP 2 081 619 B1**
(45) Date of publication and mention of the grant of the patent: **23.11.2016**
(21) Application number: 07861406.2
(22) Date of filing: 15.10.2007
(51) Int. Cl.: A61M 1/06

(54) **CONNECTOR FOR USE IN SINGLE AND DOUBLE BREAST PUMPING**
VERBINDUNGSGLIED ZUR VERWENDUNG IN EINZEL- UND DOPPEL-BRUSTPUMPEN
RACCORD UTILISÉ LORS DU TIRAGE DU LAIT AU NIVEAU D'UN SEIN OU DES DEUX SEINS

(30) Priority: 13.10.2006 US 580465
(43) Date of publication of application: 29.07.2009
(73) Proprietor: Medela Holding AG, 6340 Baar (CH)
(72) Inventor: LUZBETAK, Mark A., Kildeer, IL 60047 (US); SUTRINA, Thomas A., Rockford, IL 61108 (US)
(74) Representative: Clerc, Natalia
(86) International application number: PCT/US2007/021973
(87) International publication number: WO 2008/048535

(56) References cited:
- US-A- 4 566 480
- US-A- 5 333 606
- US-A- 5 720 722
- US-A1- 2004 127 845
- US-A1- 2004 127 845

## Description

### FIELD OF THE INVENTION

The present invention relates to breastpumps, and more specifically to a breastpump system or assembly which can be used in both single and double pumping modes of operation. In particular, the invention includes a connector, which permits a variety of configurations to enable use of a breastpump in either single or double pumping modes.

### BACKGROUND OF THE INVENTION

Breastmilk pumps are well-known, and generally comprise a hood, or shield, which fits over the breast. A vacuum pump is connected to the shield for generating an intermittent vacuum (i.e., negative pressure) within the shield. A receptacle is provided in communication with the shield for receiving the breast milk expressed during operation of the pump.

The action of the pump creates an intermittent vacuum within the shield, which serves to create an environment reminiscent of suckling and thus, causes expression of breast milk from the mother's breast. The milk so expressed is ordinarily collected in a bottle or other container for storage and later use. Such breast pumps are disclosed in U.S. Patent Nos. 4,857,051; 5,007,899; and 5,071,403 for example.

It is also well-known to provide a breast pump which can be used in both single (one breast) and double (both breasts) modes of operation, i.e., expressing breast milk from one breast at a time or simultaneously. For example, Medela, Inc., to which the present invention is assigned, provides a multi-port connector for use with its commercialized CLASSIC vacuum pump apparatus. In a double-pumping mode, two airlines connect to a pair of ports in a connector, which in turn is connected to a pump. The airlines are individually connected to two breast pump assemblies (to convey changes in pressure to the connected breast shields). Two adapters are further provided, each containing a milk barrier to prevent milk from reaching the vacuum pump. Each adapter is releasably attached to a respective breast pump assembly through a threaded engagement. This arrangement using the connector including two adapters of the foregoing type is disclosed in U.S. Patent No. 5,071,403.

With the above noted configuration, single pumping is achieved by removing one of the tubes from the connector to disconnect the breastshield. A plug is inserted into the joint or receptacle from which the tube was removed so that suction is achieved only in the remaining breastshield. In using this design, the user has to disconnect the tube from both the shield and the joint and plug the joint to inactivate one of the two breast shields. The suction level for single pumping can be higher due to the loss in fluid volume compared with two breast shields. The removed tubing can be misplaced or lost.

U.S. Patent No. 5,720,722 is a connector for use in single and double breast pumping. This connector has a tubular housing with an internal wall extending across the housing interior to divide it into two chambers. One chamber has an outlet for attachment to an air tube for single breast pumping, while the second chamber has two outlets for attachment to two air tubes for double breast pumping.

In this configuration, when a woman wants to only pump one breast, the tube from the inactive breastshield is removed from the breast pump and a plug is inserted into the open port of the pump to close it off. The plug may also provide for a predetermined amount of air leakage to simulate the load of the disconnected breastshield, so that the single shield pumping suction level is substantially the same to the double pumping vacuum level. However, the user may accidentally tangle the two tubes, either during storage, setup, or use. Tangled tubes can also lead to a nursing mother accidentally disconnecting the incorrect breastshield when single breast pumping is desired.

Thus, a breastpump that eliminates a significant number of connections and attachment parts for transition between single and double breast pumping would be considered a desired improvement in the art, thereby facilitating easier use of a breast pump and yielding fewer parts for the user to carry, clean, misplace or manipulate.

US 5333 606 discloses an accessory adaptor for a respirator, wherein this adaptor has to be plugged into an adaptor port of a manifold and wherein this accessory adaptor comprises a leakage path.

US 4 566 480 discloses a connector according to the preamble of claim 1.

### SUMMARY OF THE INVENTION

An aspect of the present invention eliminates a significant number of attachment parts, and the need for adapters, to provide a breast pump which uses fewer connections and attachments, thereby facilitating easier use of the breast pump and yielding fewer pieces for the user to carry, clean, misplace or manipulate.

A further objective is to provide a connector that is readily used in either a single or double pumping mode through an easy and effective engagement of a fluid conveying device.

An aspect of the invention provides a connector according to claim 1, this connector including a manifold. The manifold includes a three-way passageway formed therethrough. The manifold includes a first port, a second port, a third port and a fourth port. The first, second and third ports are in fluid communication with the three-way passageway. A first tube is connected to and in fluid communication with the first port. A second tube is connected to and in fluid communication with the second port and a third tube is connected to and in fluid communication with the third port. The third tube is sized and shaped to be connectable to a vacuum source, and the fourth port is sized and US 4566 480 discloses a connector according to the preamble of claim 1. shaped to receive one of the first tube and the second tube and permit a predetermined amount of fluid to pass by way of a leakage path. The leakage path is formed in the manifold.

Preferred embodiments of the invention include a plug member that terminates each tube. The first tube terminates with a first plug member, the second tube terminates with a second plug member and the third tube terminates with a third plug member. The third plug member is sized and shaped to be connectable to a vacuum source and the fourth port is sized and shaped to receive one of the first plug member and the second plug member.

Another preferred embodiment includes a plug member within the fourth port of the manifold. One of the first tube or second tube is adapted to connect to the plug member of the fourth port.

Other preferred embodiments of the invention provide the first, second and third tubes non-removably connected to the manifold. The fourth port may include an O-ring disposed therein, the O-ring including a pathway formed thereon for permitting air to be drawn therepast. The three-way passageway may be a T-shaped or Y-shaped passageway formed in the manifold. The plug members may engage with a respective one of the ports in a substantially fluid tight fit.

Another embodiment of the invention provides a system for breastpumping in a single breastpumping mode or a double breastpumping mode, including a source of intermittent vacuum. First and second breast shields are connectable to the source of vacuum. A connector according to claim 1 is provided which includes a connector body having four spaced ports, a first tube extending from a first of the four ports with a first tube end adapted to connect to the source of intermittent vacuum. A second tube extends from a second of the four ports with a second tube end adapted to connect to one of the first and second breast shields. A third tube extends from a third of the four ports with a third tube end adapted to connect to the other of the first and second breast shields. A passageway is formed in a central body of the connector, the passageway fluidly connecting the first, second and third tubes, and a fourth of the four ports adapted to receive one of the second and third tube ends and functioning to substantially seal the received tube end.

Yet other preferred embodiments of the invention provide the first, second and third tubes being non-removably connected to the connector body. The passageway may be a three-way passageway. The passageway may be a T-shaped or Y-shaped passageway. The fourth of the four ports may be substantially sealed and provided with a predetermined amount of leakage.

It will of course be understood that the aspects and objectives of the invention are various, and need not be all present in any given embodiment of the invention. The features, advantages and accomplishments of the invention will be further appreciated and understood upon consideration of the following detailed description of an embodiment of the invention, taken in conjunction with the drawings, in which:

### BRIEF DESCRIPTION OF THE DRAWINGS

The foregoing objectives and advantages of the invention will be further understood upon consideration of the following detailed description of an embodiment of the invention taken in conjunction with the drawings, in which:
FIG. 1 is a diagrammatic drawing of a breast pump assembly incorporating a connector for use in single and double breast pumping according to one embodiment of the present invention;
FIG. 2 is a perspective view of a connector according to one embodiment of the present invention in a single pumping configuration or mode;
FIG. 3 is a perspective view of the connector of FIG. 2 in a double pumping configuration or mode;
FIG. 4 is a cross-sectional view of a manifold and connected terminal fastener or plug for the connector according to one embodiment of the present invention;
FIG. 5 is a cross-sectional view of the manifold of the connector according to the present invention;
FIG. 6 is an exploded perspective view of an embodiment of a tube connector;
FIG. 7 is a perspective view of the receptacle portion of the tube connector of FIG. 6
FIG. 8 is a perspective view of the plug member of the tube connector of FIG. 6;
FIG. 9 is a perspective view of the latching component of the tube connector of FIG. 6;
FIG. 10 is an assembled cross-sectional view of the tube connector of FIG. 6;
FIG. 11 is an exploded view of the tube connector of FIG. 6 and a motor drive unit;
FIG. 12 is an exploded sectional view of the tube connector of FIG. 6 and a motor drive unit;
FIG. 13 is an assembled sectional view of the tube connector of FIG. 6 and a motor drive unit;
FIG. 14 is an exploded perspective view of an alternate embodiment of a tube connector;
FIG. 15 is an exploded sectional view of the alternate embodiment of the tube connector of FIG. 14;
FIG. 16 is a perspective view of the latch portion of the alternate embodiment of the tube connector of FIG. 14;
FIG. 17 is a perspective sectional view of the receptacle of the alternate embodiment of the tube connector of FIG. 14;
FIG. 18 is a perspective view of the plug member of the alternate embodiment of the tube connector of FIG. 14;
FIG. 19 is an assembled sectional view of the alternate embodiment of the tube connector of FIG. 14;
FIG. 20 is an exploded sectional view of yet another alternate embodiment of a tube connector;
FIG. 21 is a perspective sectional view of the receptacle of an alternate embodiment of the tube connector of FIG. 20;
FIG. 22 is a perspective view of a latch portion of an alternate embodiment of the tube connector of FIG. 20;
FIG. 23 is an assembled sectional view of the alternate embodiment of the tube connector of FIG. 20;
FIG. 24 is a perspective sectional view of an alternate embodiment of a tube connector; and
FIG. 25 is an assembled sectional view of the alternate embodiment of the tube connector of FIG. 24.

### DETAILED DESCRIPTION OF AN EMBODIMENT OF THE INVENTION

The connector of the present invention will be described herein in use with a breastpump assembly, but it is contemplated that the connector of the present invention can be used in any device that may benefit from this type of connector.

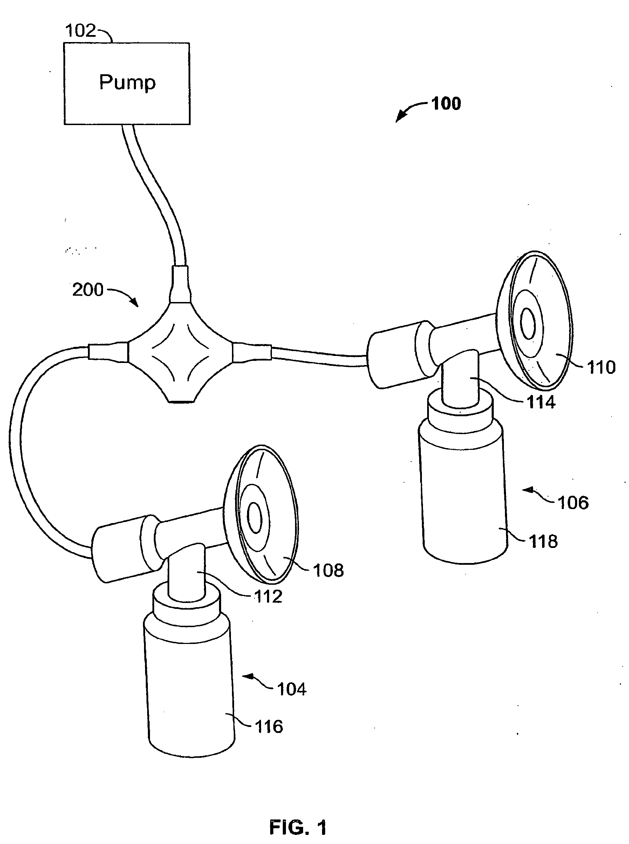

A breastpump assembly 100' for pumping two breasts simultaneously or a single breast shows, in FIG. 1, a vacuum pump 102', which is used to generate a periodic change in pressure or intermittent vacuum. The generated change in pressure from the pump 102' is then transmitted through connector 200'. The connector 200' may be configured to supply changes in pressure to breastshield assemblies 104', 106' simultaneously or only one of the two assemblies.

As referred to herein, vacuum is meant to denote a pressure less than ambient and in one configuration of the connector 200' is supplied to the breast shields 108', 110', and through the breast shields applied to a breast placed therein to express milk. Reference to a tube or passage hereafter as an "air" tube or a "vacuum" tube is not intended to be limiting.

The breast shield assemblies 104', 106' may each include, in addition to the shields 108', 110', a respective conduit structure 112', 114'. Each respective conduit structure 112', 114', is typically provided in fluid communication with a collecting container, a bottle or the like, 116', 118'.

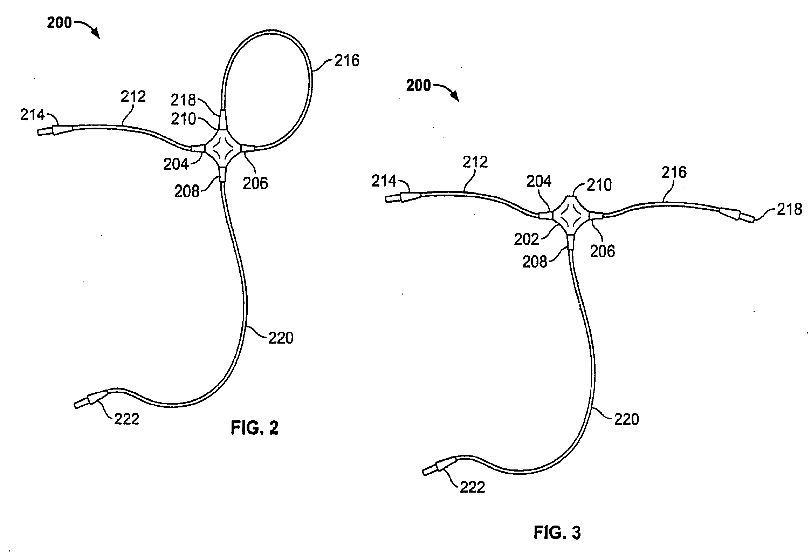

FIGS. 2 and 3 illustrate a connector 200' according to the illustrated embodiment of the present invention in a single pumping and a double pumping configuration, respectively. The connector 200' includes a manifold 202'. The manifold 202' is a central body including four spaced receptacles or ports, 204', 206', 208', and 210'.

The connector 200' includes three tubes, a first tube 212', a second tube 216' and a third tube 220'. A first tube 212' is connected to and extends from port 204' and may further include a first plug member 214' at a terminal end thereof. A second tube 216' is connected to and extends from port 206' and may further include a second plug member 218' at a terminal end thereof. A third tube 220' is connected to and extends from port 208' and may further include a third plug member 222' at a terminal end thereof. Tubes 212', 216', 220' are preferably permanently affixed within ports 204', 206', 208' such that tubes 212', 216', 220' are not detachable from the manifold, which is considered one advantage over the prior art.

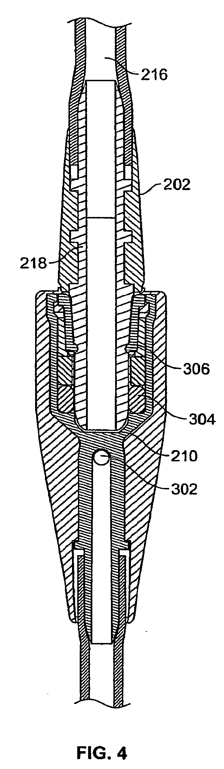

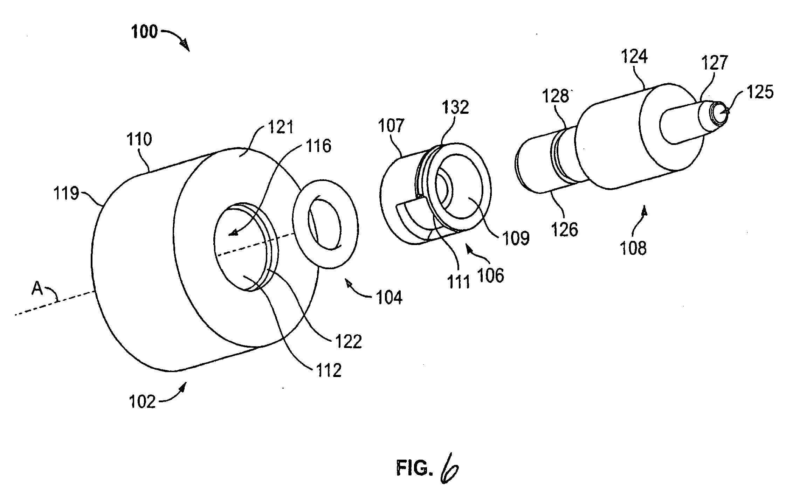

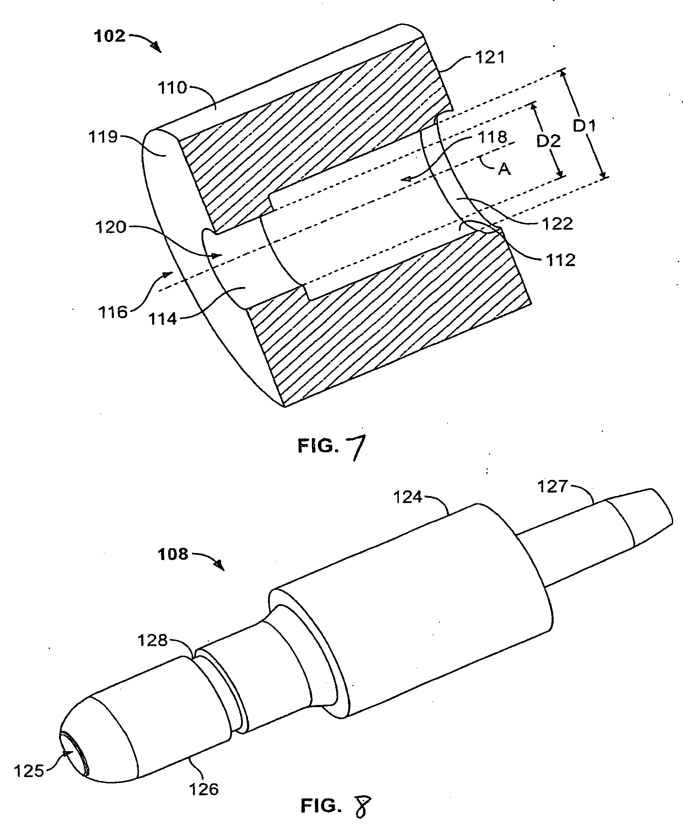

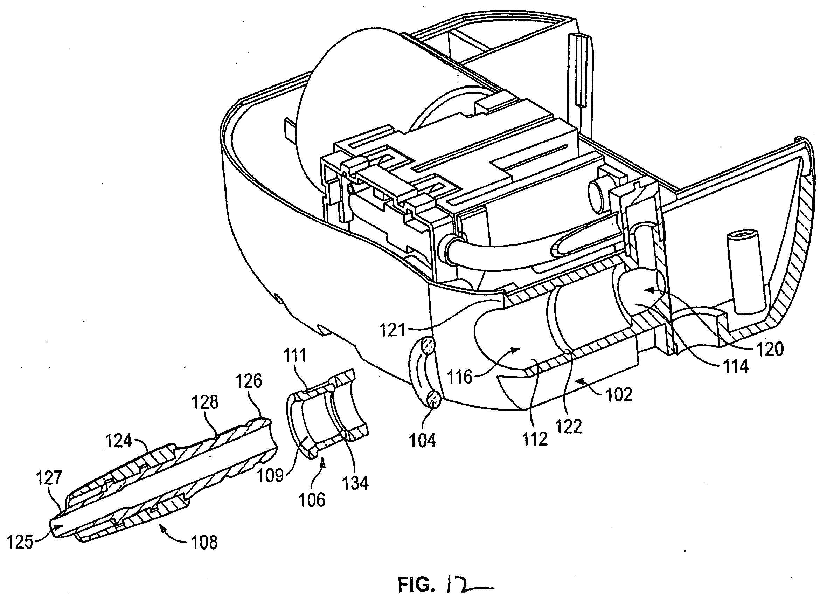

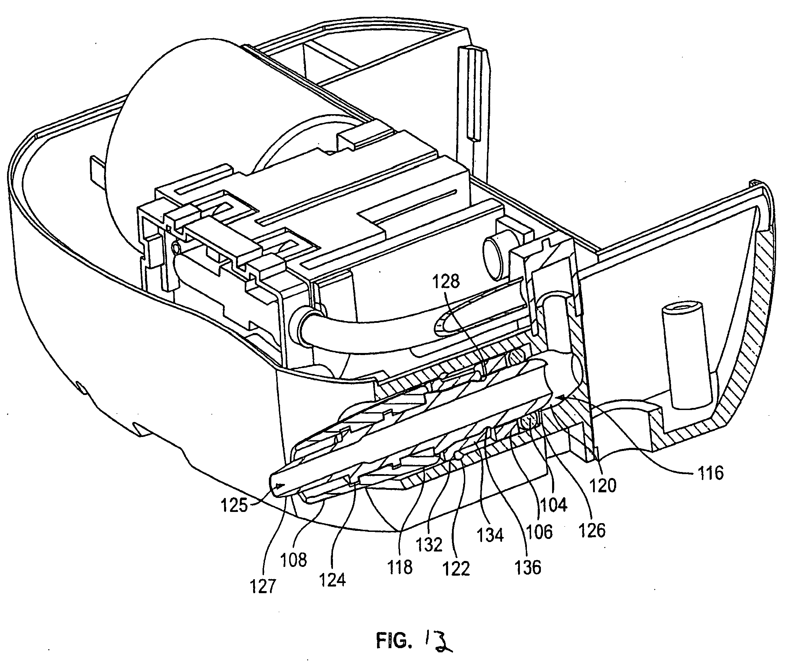

The port or dock port 210' includes a tube connector arrangement (see FIG. 4), as disclosed and described in FIGS. 6-25. FIG. 6 is an exploded view of a first embodiment of a tubing connector 100. The tubing connector 100 includes a receptacle 102, a sealing ring 104, a latching component 106 and a plug member 108. As shown in FIG. 7, receptacle 102 has an exterior surface 110, a first interior surface 112 and a second interior surface 114, the first and second interior surfaces 112, 114 define a bore 116 therethrough.

It will be understood and seen herein, that the receptacle 102 is not necessarily a separate piece as depicted, but is more typically made integral with something within which the plug 108 is to connect. Receptacle 102 may therefore be a piece that is assembled to, for example, a motor drive as shown hereafter, or could be formed integral therewith. "Receptacle" is therefore used to generally also refer to a well, socket, orifice and the like.

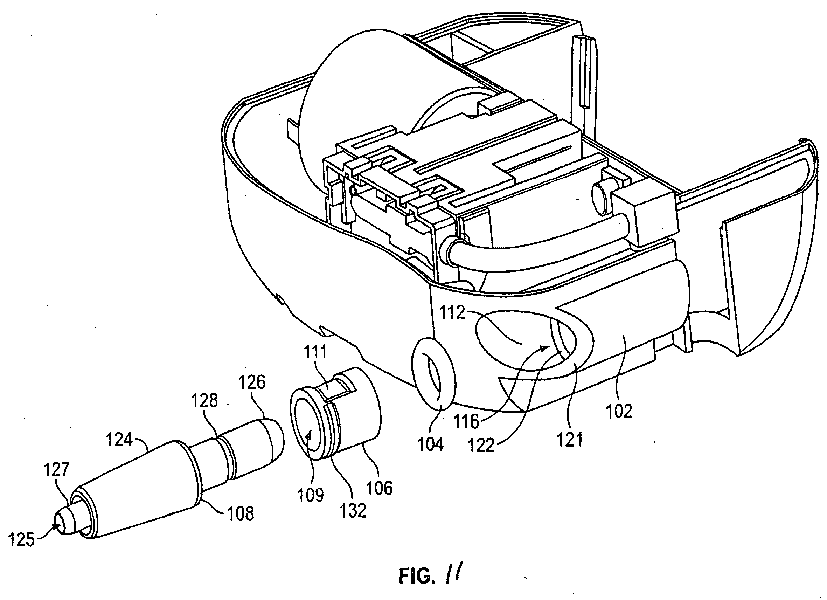

Referring to FIG. 7, an axis A extends into bore 116. Bore 116 has a first portion 118 defined by first interior surface 112 and a second portion 120 defined by second interior surface 114. First portion 118 has a first diameter D1 and second portion 120 has a second diameter D2. The first interior surface 112 further includes a circumferential groove or channel 122. Receptacle 102 includes two ends 119, 121. The first end 119 of receptacle 102 is adapted, for instance, to be connected to a motor drive unit of a breast pump assembly, or formed integrally therewith, as shown in FIGS. 11-13. The second end 121 of receptacle 102 receives other components of a tube connector therein, as will be described in detail below.

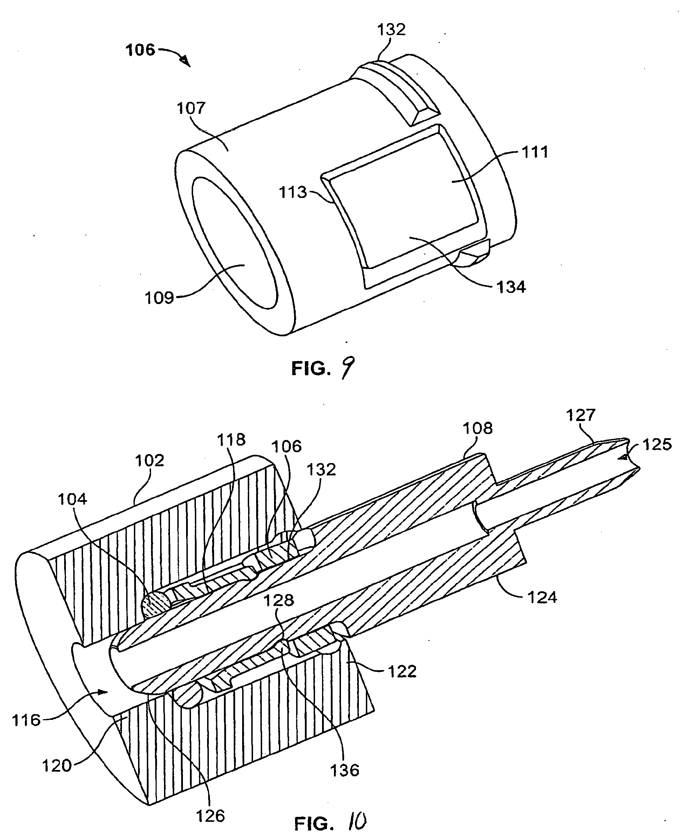

As shown in FIG. 8, plug member 108 has a base 124 and a stem 126. The base 124 is adapted to be attached to a tube (not shown) via a nipple 127. The stem 126 extends axially from the opposite end of the base 124. A passageway 125 extends through the plug member 108 between the base 124 and the stem 126 for conveying fluid/air. The stem 126 has a circumferential groove or channel 128 therein, and is adapted to be received within the latching component 106 (see FIG. 9). Latching component is preferably formed of an elastomeric material allowing deflection for securing parts of the tubing connector and providing a seal therebetween.

The latching component 106 of FIG. 9 is in the form of a sleeve sized and shaped to receive the stem 126 of plug member 108 therein, and in turn to be received in the first portion 118 of bore 116 of receptacle 102. Latching component 106 includes an interior surface 109 and exterior surface 107, exterior surface 107 having a circumferential ridge 132 thereon; here, one ridge 132 does not go completely around the circumference. Latching component 106 further includes two resilient tabs 134 spaced diametrically opposite one another, although any number of tabs is contemplated. The tabs 134 are connected (e.g., made integral) at one end 111 to the latch component 106 in a hinge-like arrangement. The free end 113 of each resilient tab 134 has a rib 136 (FIG. 10) extending past the inside surface 109 and slightly within the interior of the latching component 106. The ridge 132, which is a partial ring, engages with the groove 122 of the first interior surface 112 of the receptacle 102 when the latching component 106 is inserted into the first portion 118 through bore 116 of receptacle 102. The rib 136 is arranged to resiliently engage with circumferential channel 128 on stem 126 of plug member 108 when inserting plug member 108 into latching component 106.

One manner of assembly of the receptacle 102, sealing ring 104, latching component 106 and plug member 108 is shown in FIG. 10. Sealing ring 104 is inserted axially into the first portion 118 of bore 116 of the receptacle 102. The sealing ring 104 has an outer diameter D 1 that fits within first portion 118 of bore 116, and greater than D2 of second portion 120 of bore 116 such that sealing ring 104 abuts the second portion 120 of bore 116. The latching component 106 is then inserted into the first portion 118 of bore 116 abutting sealing ring 104 forming an airtight seal, and circumferential ridge 132 engages with the groove 122 of the first interior surface 112 of the receptacle 102. The latching component 106 is secured within receptacle 102 in a manner such that the latching component 106 does not move axially within receptacle 102, although it is contemplated that the latching component 106 and receptacle 102 could have a rotatable engagement. The stem 126 of the plug member 108 is inserted into latching component 106. The ribs 136 on tabs 134 resiliently engage with the circumferential channel 128 on stem 126 which secures the plug member 108 within latching component 106 and thus within receptacle 102. The engagement between ribs 136 and the circumferential channel 128 is such that the plug member 108 can rotate generally freely within the latching component 106. Latching component is preferably formed of a plastic material.

As shown in FIGS. 11-13, receptacle 102 can be formed integrally with the motor drive unit of a breast pump assembly. Assembly of the tube connector 100 in this embodiment is similar to that described above. Sealing ring 104 is inserted axially into the first portion 118 of bore 116 of the receptacle 102 such that sealing ring 104 abuts the second portion 120 of bore 116. The latching component 106 is inserted as previously described with circumferential ridge 132 engaging with the groove 122, securing the latching component 106 within receptacle 102. Plug member 108 can then be releasably inserted into latching component 106, with tabs 134 resiliently engaging with the circumferential channel 128 on stem 126, to secure the plug member 108 within latching component 106 and thus within receptacle 102.

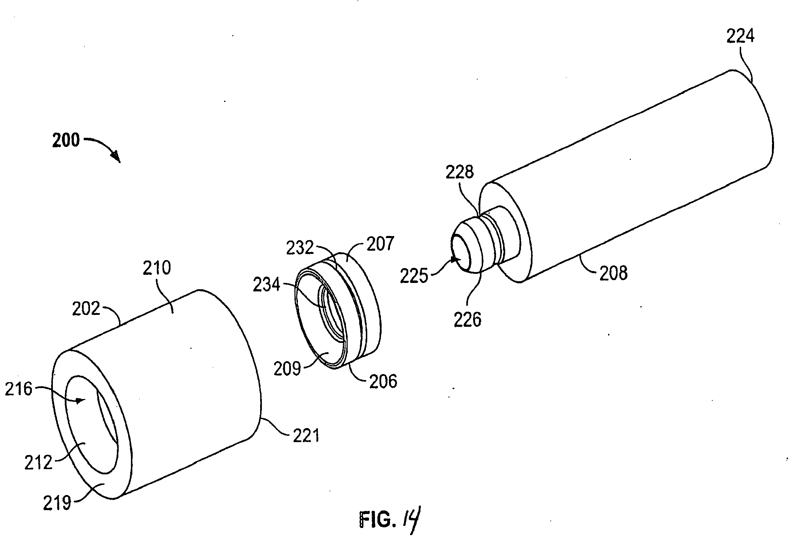

FIG. 14 is an exploded view of a second embodiment of a tubing connector 200. This second embodiment tubing connector 200 includes a receptacle 202, a latching component 206 and a plug member 208.

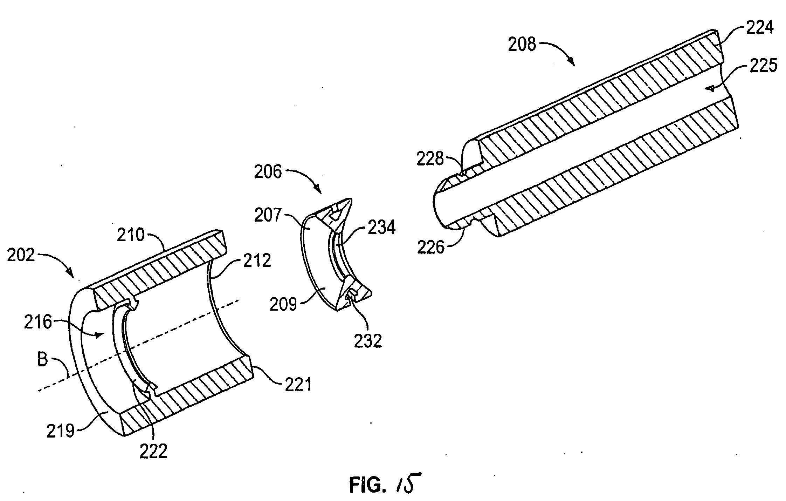

As shown in FIG. 15, this receptacle 202 has an exterior surface 210 and an interior surface 212, interior surface 212 defining a bore 216 therethrough. An axis B is defined extending into bore 216. The interior surface 212 has a ring or protrusion 222 thereon around its entire circumference. Receptacle 202 includes two ends 219, 221. The first end 219 of receptacle 202 is adapted, for instance, to be connected to a motor drive unit of a breast pump assembly, or formed integrally therewith. The second end 221 of receptacle 202 receives other components of tube connector 200 therein as will be described in detail.

Also shown in FIG. 15, plug member 208 is adapted at one end 224 to be attached to a tube (not shown). The tube could then be formed integral therewith, or otherwise connected to passageway 225. A stubby stem 226 extends axially from the opposite end of base 224. Passageway 225 extends through the plug member 208 between the end 224 and the stem end 226 for conveying fluid/air. The stem 226 has a circumferential groove or channel 228 therein adapted to be received within the latching component 206.

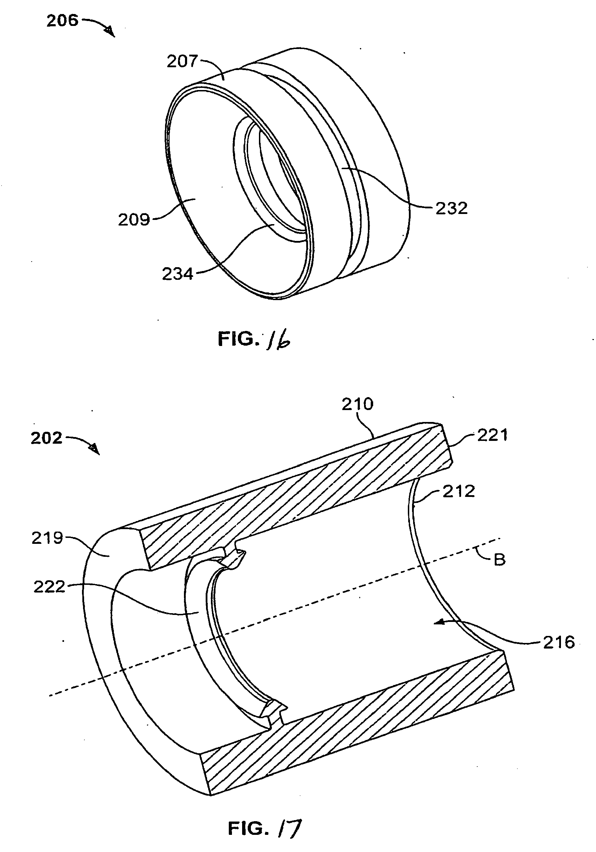

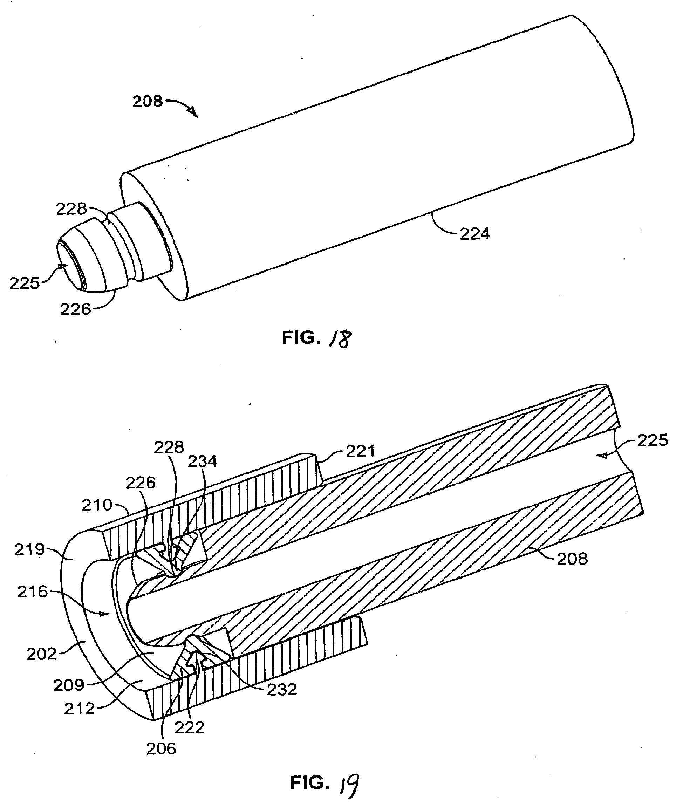

Referring to FIG. 16, this latching component 206 is likewise in the form of a sleeve sized and shaped to receive the stem 226 of plug member 208 therein, and to be received in the bore 216 in this type of receptacle 202. Modified latching component 206 includes an interior surface 209 and exterior surface 207. Exterior surface 207 has a circumferential groove or channel 232 thereon that engages with the ring 222 on the interior surface 212 of the receptacle 202 (FIG. 17) when the latching component 206 is inserted into the bore 216 in receptacle 202. Latching component 206 further includes a rib or ridge 234 on its interior 209 sized and shaped to match (FIG. 18) the circumferential groove 228 of stem 226 of plug member 208, such that when plug member 208 is inserted into latching component 206, rib 234 engages circumferential groove 228, which rotatably secures plug member 208 within latching component 206.

This is shown in FIG. 19, where latching component 206 is inserted axially into the bore 216, and groove 232 engages with the protrusion 222 on the interior surface 212 of the receptacle 202 which secures the latching component 206 within receptacle 202. The stem 226 of the plug member 208 is inserted into latching component 206 and the rib 234 engages with the circumferential groove 228 on stem 226 which secures the plug member 208 within latching component 206 and thus within receptacle 202. The engagement between the rib 234 and the circumferential groove 228 is such that the plug member 208 can rotate freely within the latching component 206. Latching component is preferably formed of an elastomeric material allowing deflection for securing parts of the tubing connector and providing a seal therebetween.

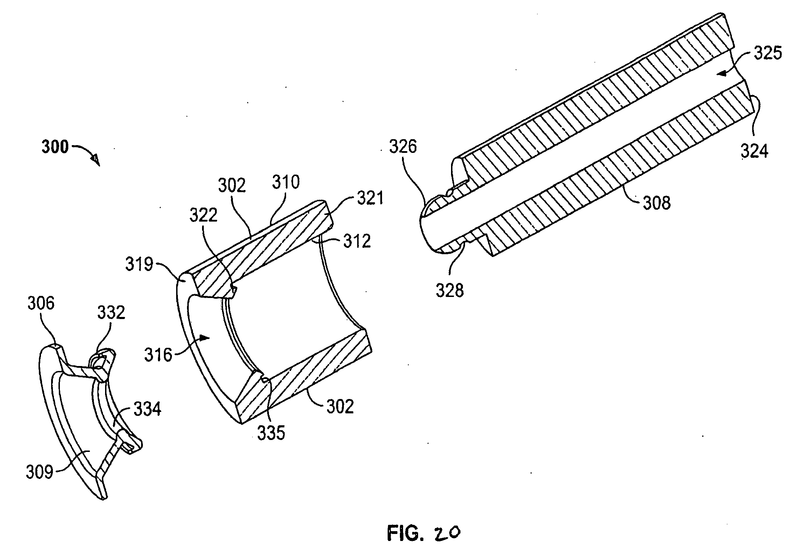

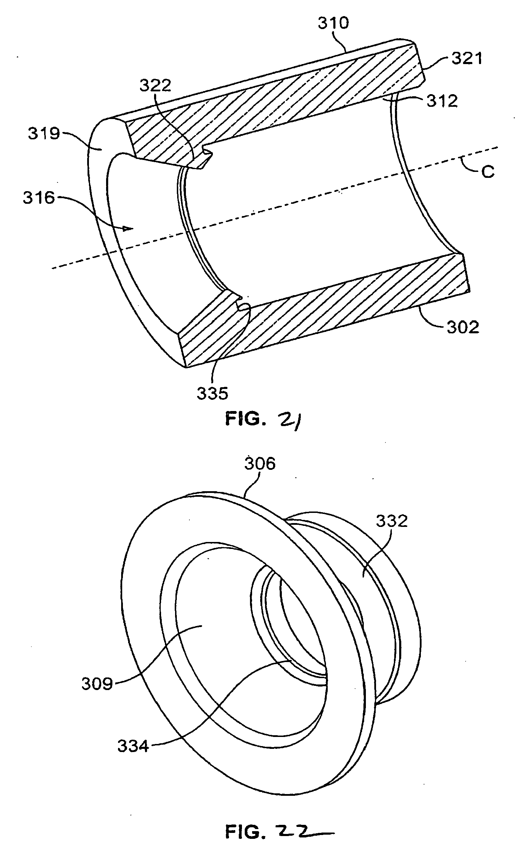

FIG. 20 is an exploded view of a third embodiment of a tubing connector 300. This third embodiment tubing connector 300 includes a receptacle 302, a latching component 306 and a plug member 308. As shown in FIG. 21, receptacle 302 has an exterior surface 310 and an interior surface 312, interior surface 312 defining a bore 316 therethrough. An axis C is defined extending into bore 316. The interior surface 312 has a circumferential lip 322 thereon around its entire circumference. Receptacle 302 includes two ends 319, 321. The first end 319 of receptacle 302 is adapted, for instance, to be connected to a motor drive unit of a breast pump assembly, or formed integrally therewith. The second end 321 of receptacle 302 receives other components of tube connector 300 therein as will be described in detail.

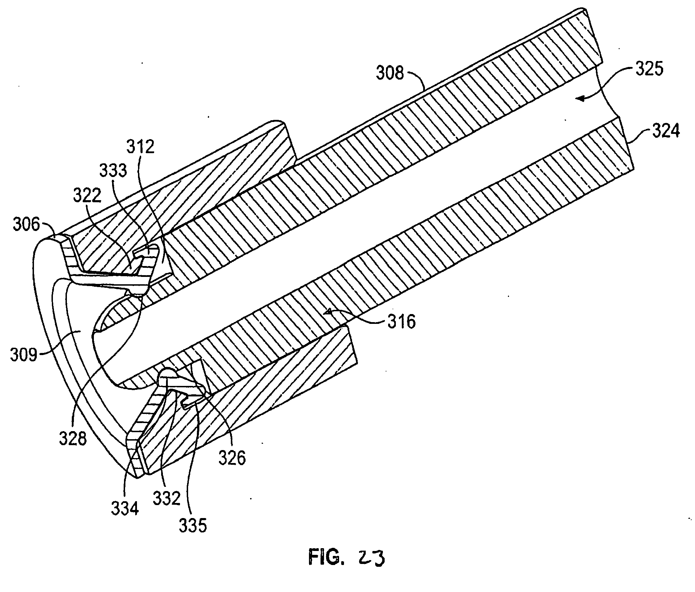

Turing to FIG. 22, latching component 306 includes an interior surface 309 and a circumferential groove or channel 332 on the outboard side. Groove 332 is annular in form, being located beneath an overlying lip 333. Latching component 306 further includes a shoulder 334 on its interior 309 sized and shaped to match the circumferential groove 328 (FIG. 20) in stem 326 of plug member 308 such that when plug member 308 is inserted into latching component 306 (FIG. 23), shoulder 334 engages circumferential groove 328, which secures plug member 308 within latching component 306.

As further shown in FIG. 23, plug member 308 has an end 324 adapted to be attached to a tube (not shown) in any number of well known ways. A stem 326 (FIG. 20) extends axially from the opposite end of the plug member 308. A passageway 325 extends through the plug member 308 between the base 324 and the stem 326 for conveying fluid/air.

Latching component 306 is sized and shaped to receive the stem 326 of plug member 308 therein, and to be received in the bore 316 in receptacle 302. The groove 332 of latching component 306 engages with the lip 322 along the interior surface 312 of the receptacle 302, with lip 333 thereby snapping into place within a channel 335 formed between the lip 322 and adjacent sidewall of the receptacle 302 when the latching component 306 is inserted into the bore 316 in receptacle 302. This is a snap-fit between the receptacle 302 and latching component 306, and these elements are so sized to thus engage.

In one manner of assembly, latching component 306 is inserted axially into the bore 316, and groove 332 engages with the lip 322 on the interior surface 312 of the receptacle 302 which secures the latching component 306 within receptacle 302. The stem 326 of the plug member 308 is inserted into the combined latching component 306 and receptacle 302, and the shoulder 334 engages with the circumferential groove 328 on stem 326, thus securing the plug member 308 within latching component 306 and within receptacle 302. The engagement between the shoulder 334 and the circumferential groove 328 is such that the plug member 308 can rotate freely within the latching component 306. Latching component is preferably formed of an elastomeric material allowing deflection for securing parts of the tubing connector and providing a seal therebetween.

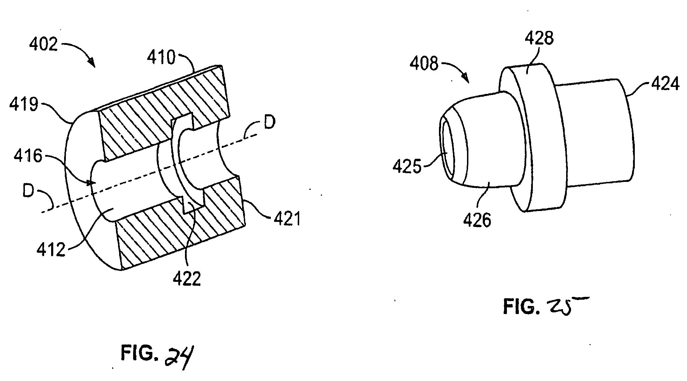

Yet another embodiment is depicted in FIGS. 24-25. As shown in FIG. 24, receptacle 402 has an exterior surface 410, and an interior surface 412. The interior surface 412 defines a bore 416 therethrough. An axis D extends into bore 416. A circumferential groove 422 is formed around the interior surface 412. Receptacle 402 includes two ends 419, 421. The first end 419 of receptacle 402 is adapted, for instance, to be connected to a motor drive unit of a breast pump assembly, or formed integrally therewith. The second end 421 of receptacle 402 receives other components of yet another tube connector therein, as will be described in detail below.

The plug member 408 has an end 424 and a stem 426. The end 424 is adapted to be attached to a tube (not shown) in any of many known ways. The stem 426 extends axially from the opposite end of the base 424. A passageway 425 extends through the plug member 408 between the end 424 and the stem 426 for conveying fluid/air.

As further shown in FIG. 25, sealing member 404 is sized to fit in the circumferential groove 422 of the interior surface 412 of the receptacle 402. This seal member 404 engages the circumferential groove 428 of the plug member 408 when plug member 408 is inserted into the bore 416 of the receptacle 402 thereby forming an air-tight seal, acting to retain the plug member 408 within the receptacle 402 by resisting removal forces, and also allowing the plug member 408 to rotate freely within the receptacle 402. It will be understood that the receptacle 402 could be integral with a housing and further include a shoulder surface on the interior 412, similar to embodiments previously described, for engagement with a plug member. Furthermore, a component retained within the bore, or a surface integral with the receptacle, could define and function as a sealing member. There are many contemplated embodiments that serve to simplify fabrication and assembly.

Returning to FIGS. 1-5, the port 210' permits connection and disconnection of either tube 214', 216' when single breast pumping is desired. More particularly, port 210' permits connection and disconnection of either of plug members 214', 218' when single breast pumping is desired.

Plug member 222', when engaged with an operating vacuum pump transmits changes in pressure from the pump to the manifold 202' and through tubes 212' and 216' to one or both of the breastpump assemblies. When single breast pumping is desired, one of either tube 212' or tube 216' is disconnected from the respective breastpump assembly and docked or engaged with port 210'. The plug 214'or 218' (depending on which single breast a nursing mother wishes to pump) engages with the tube connector arrangement within dock port 210' and changes in pressure generated by the pump are conveyed through the manifold 202' to the tube not connected to dock port 210'. Likewise, the dock port 210' can include a plug member such that either tube 212' or tube 216' can connect thereto.

As shown in the cross-sectional view of the manifold of FIG. 4, the tube connector arrangement within port 210' includes a sealing ring 304' and latching component 306', again fully described herein and shown in FIGS 6-25, for engagement with plug connector 214' or 218' (here, 218'). The manifold 202' includes a three-way, or T-shaped or Y-shaped, flow passage 302' to convey the vacuum from the pump to the connector 200' and into separate air paths via tube 212' and/or 216' depending on whether single or double breast pumping is implemented. The passage 302' is not connected to port 210'.

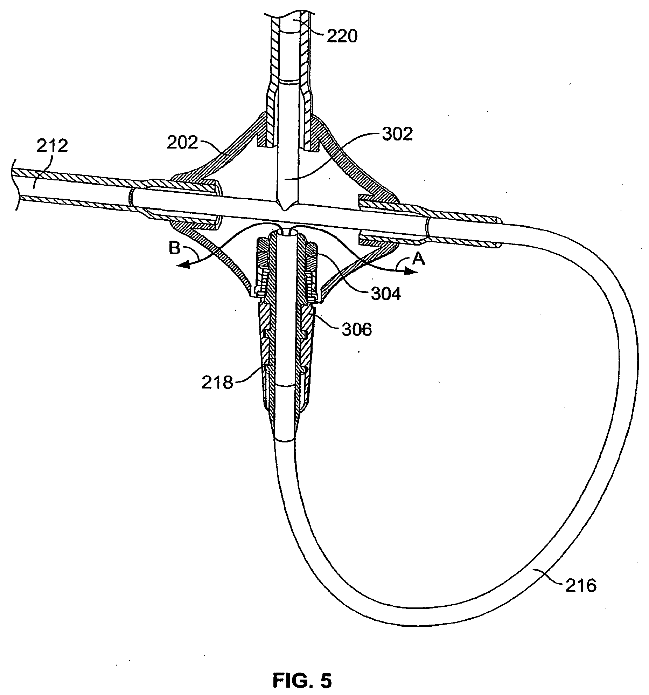

If double breast pumping is desired, plug member 214' connects tube 212' to a first breastpump assembly and plug member 218' connects tube 216' to a second breastpump assembly. Port 210' does not need to be plugged or capped off since the T-shaped flow passage 302' (see FIG. 5) conveys pressure changes to each tube 212', 216'. When single breast pumping is desired, plug connector 218' is engaged with port 210'. Alternatively, plug connector 214' can engage with port 210' depending on which breast the mother desires to pump.

Once plug connector 218' engages with port 210', port 210' provides a predetermined amount of air leakage to simulate the load of the disconnected breastshield, so that the single shield pumping suction level is substantially the same to the double pumping vacuum level. As shown in FIG. 5, two air leakage paths A, B are formed in port 210' such that the disconnected breastshield does not, to a great extent, adversely affect vacuum levels transmitted to the operating breastshield. Path A leaks air past both the sealing ring 304' and latching component 306' whereas path B leaks air past the sealing ring 304' and between the latching component 306' and plug member 218'. The leaked air travels into from outside the manifold 202'. The leaked air may take any path, including a path through the manifold itself.

It is understood that all shapes and sizes, configurations of the tube connector are considered various embodiments thereof. It is seen that the objects set forth above, among those made apparent from the preceding description, are efficiently attained and, since certain changes may be made in the above constructions without departing from the scope of the invention, it is intended that all matter contained in the above description or shown in the accompanying drawings shall be interpreted as illustrative and not in a limiting sense. It is also to be understood that the following claims are intended to cover all of the generic and specific features of the invention herein described and all statements of the scope of the invention that, as a matter of language, might be said to fall there between.

## Claims

1. A connector (200), especially for use with a system for single or double breastpumping, comprising,
a manifold (202), said manifold including a three-way passageway formed therethrough, said manifold (202) including a first port (204), second port (206), third port (208) and fourth port (210), said first, second and third ports (204, 206, 208) being in fluid communication with said three-way passageway, and fourth port (210) not being in fluid communication with said three-way passageway;
a first tube (212) connected to and in fluid communication with said first port (204), a second tube (216) connected to and in fluid communication with said second port (206);
and
a third tube (220) connected to and in fluid communication with said third port (208), wherein said third tube (220) is sized and shaped to be connectable to a vacuum source,
**characterized in that**, said first tube (212) terminates with a first plug member (214), said second tube (216) terminates with a second plug member (218), said fourth port (210) is sized and shaped to receive one of said first plug member (214) and said second plug member (218) to permit a predetermined amount of fluid to pass by way of a leakage path (A, B) when received, wherein said leakage path (A, B) is formed in said manifold (202).

2. The connector of Claim 1, wherein the third tube (220) terminates with a third plug member (222).

3. The connector of Claim 2, wherein each of said first and second plug members (214, 218) are adapted to connect with said fourth port (210) to substantially seal said plug members (214, 218) with said fourth port (210).

4. The connector of Claim 2 wherein said third plug member (222) is sized and shaped to be connectable to a vacuum source (102).

5. The connector of Claims 1 or 4, wherein said first, second and third tubes (212, 216, 220) are non-removably connected to said manifold (202).

6. The connector of Claims 1 or 4, wherein said fourth port (210) includes an O-ring disposed therein, said O-ring including a pathway (302) formed thereon for permitting air to be drawn therepast.

7. The connector of Claims 1 or 4, wherein said three-way passageway (302) is a T-shaped or Y-shaped passageway formed in said manifold (202).

8. The connector of Claim 1, wherein the leakage path (A, B) is formed in the fourth port (210).

9. A system for breastpumping in a single breastpumping mode or a double breastpumping mode, comprising:
a source of intermittent vacuum (102);
a first breast shield (108);
a second breast shield (110); and
a connector (200) according to one of claims 1 to 8, including a connector body having said four ports (204, 206, 208, 210) being spaced from each other, said first tube (212) extending from said first (204) of said four ports with said first tube end (214) adapted to connect to the other of said first and second breast shields (108, 110), said second tube (216) extending from said second (206) of said four ports with said second tube end (218) adapted to connect to one of said first and second breast shields (108, 110), said third tube (220) extending from said third (208) of said four ports with said third tube end (222) adapted to connect to said source of intermittent vacuum (102), said passageway formed in a central body of said connector (200), said passageway fluidly connecting said first, second and third tubes (212, 216, 220), and said fourth (210) of said four ports adapted to receive one of said first and second tube ends and functioning to substantially seal said received tube end and permit a predetermined amount of fluid to pass by way of leakage of a leakage path (A, B), wherein said leakage path is formed in said fourth port (210).

10. The system of Claim 9, wherein said first, second and third tubes (212, 216, 220) are non-removably connected to said connector body.

11. The system of Claim 9, wherein said passageway is a T-shaped or Y-shaped passageway.

12. The system of Claim 9, wherein said fourth (210) of said four ports is substantially sealed and provided with a predetermined amount of leakage.

## Patentansprüche

1. Konnektor (200), insbesondere zur Verwendung in einem System für Einzel- oder Doppelbrustpumpen, aufweisend,
einen Verteiler (202), wobei dieser Verteiler einen durch ihn hindurch ausgebildeten Dreiwege-Durchgang beinhaltet, wobei der Verteiler (202) eine erste Öffnung (204), eine zweite Öffnung (206), eine dritte Öffnung (208) und eine vierte Öffnung (210) beinhaltet, wobei die erste, zweite und dritte Öffnung (204, 206, 208) in fluidkommunizierender Verbindung mit dem Dreiwege-Durchgang sind und wobei die vierte Öffnung (210) nicht in fluidkommunizierender Verbindung mit dem Dreiwege- Durchgang ist;
ein erstes Rohr (212), welches mit der ersten Öffnung (204) verbunden und in fluidkommunizierender Wirkung ist,
ein zweites Rohr (216), welches mit der zweiten Öffnung (206) verbunden und in fluidkommunizierender Wirkung ist
und
ein drittes Rohr (220), welches mit der dritten Öffnung (208) verbunden und in fluidkommunizierender Wirkung ist, wobei das dritte Rohr (220) bemessen und geformt ist, um mit einer Vakuumpumpe verbindbar zu sein,
**dadurch gekennzeichnet, dass** die erste Röhre (212) in einem ersten Steckerteil (214) endet, die zweite Röhre (216) in einem zweiten Steckerteil (218) endet, die vierte Öffnung (210) bemessen und geformt ist, um einen des genannten ersten Steckerteils (214) und des genannten zweiten Steckerteils (218) aufzunehmen und um einer vorbestimmten Fluidmenge, wenn erhalten, zu erlauben, einen Leckpfad (A, B) zu nehmen, wobei dieser Leckpfad (A, B) in diesen Verteiler (202) ausgebildet ist.

2. Konnektor gemäss Anspruch 1, wobei die dritte Röhre (220) in einem dritten Steckerteil (222) endet.

3. Konnektor nach Anspruch 2, wobei jeder des ersten und zweiten Steckerteils (214, 218) ausgebildet ist, um sich mit der vierten Öffnung (210) zu verbinden, um diese Steckerteile (214, 218) mit dieser vierten Öffnung (210) im Wesentlichen zu dichten.

4. Konnektor nach Anspruch 2, wobei der dritte Steckerteil (222) bemessen und geformt ist, um mit einer Vakuumpumpe (102) verbunden zu werden.

5. Konnektor nach einem der Ansprüche 1 bis 4, wobei die erste, zweite und dritte Röhre (212, 216, 220) unlösbar mit dem Verteiler (202) verbunden sind.

6. Konnektor nach einem der Ansprüche 1 bis 4, wobei die vierte Öffnung (210) einen in ihr angeordneten O-Ring aufweist, wobei der O-Ring einen auf ihm ausgebildeten Pfad (302) aufweist, um zu erlauben, dass Luft daran entlang geführt wird.

7. Konnektor nach einem der Ansprüche 1 bis 4, wobei der Dreiwege-Durchgang (302) ein T-förmiger oder Y-förmiger, im Verteiler (202) ausgebildeter Durchgang ist.

8. Konnektor nach Anspruch 1, wobei der Leckpfad (A, B) in der vierten Öffnung (210) ausgebildet ist.

9. System zum Brustpumpen in einem Einzelbrustpump-Modus oder einem Doppelbrustpump-Modus, aufweisend:
eine intermittierende Vakuumquelle (102);
eine erste Brusthaube (208);
eine zweite Brusthaube (110); und
einen Konnektor (200) gemäss einem der Ansprüche 1 bis 8, welcher einen Konnektorkörper aufweist, der vier voneinander beabstandete Öffnungen (204, 206, 208, 210) aufweist, wobei sich die erste Röhre (212) von der ersten dieser vier Öffnungen erstreckt, wobei das Ende (214) der ersten Röhre ausgebildet ist, um sich mit dem anderen der ersten und zweiten Brusthaube (108, 110) zu verbinden, wobei sich die zweite Röhre (216) von der zweiten (206) dieser vier Öffnungen erstreckt, wobei das Ende (218) der zweiten Röhre ausgebildet ist, um sich mit einem der ersten und zweiten Brusthaube (108, 110) zu verbinden, wobei sich die dritte Röhre (220) von der dritten (208) der vier Öffnungen erstreckt, wobei das Ende (222) der dritten Röhre ausgebildet ist, um sich mit der intermittierenden Vakuumquelle (102) zu verbinden, wobei der Durchgang in einem zentralen Körper des Konnektors (200) ausgebildet, ist, wobei der Durchgang die erste, zweite und dritte Röhre (212, 216, 220) miteinander fluidmässig verbindet, und wobei die vierte (210) der vier Öffnungen ausgebildet ist, um eines der Enden der ersten und zweiten Röhre aufzunehmen, und wobei sie zur im wesentlichen Dichtung der aufgenommen Röhre dient und einer vorbestimmten Menge an Fluid einen Durchgang durch einen Leckpfad (A, B) erlaubt, wobei der Leckpfad durch die vierte Öffnung (210) gebildet ist.

10. System nach Anspruch 9, wobei die erste, zweite und dritte Röhre (212, 216, 220) unlösbar mit dem Konnektorkörper verbunden sind.

11. System nach Anspruch 9, wobei der Durchgang ein T-förmiger oder Y-förmiger Durchgang ist.

12. System nach Anspruch 9, wobei die vierte (210) der vier Öffnungen im Wesentlichen dicht ist und eine vorbestimmte Menge an Leckage bietet.

## Revendications

1. Raccord (200), spécialement destiné à être utilisé avec un système de tirage du lait au niveau d'un sein ou des deux seins, comportant,
un collecteur (202), ledit collecteur comprenant un passage à trois voies formé à travers celui-ci, ledit collecteur (202) comprenant un premier orifice (204), un deuxième orifice (206), un troisième orifice (208) et un quatrième orifice (210), lesdits premier, deuxième et troisième orifices (204, 206, 208) étant en communication fluidique avec ledit passage à trois voies, et ledit quatrième orifice (210) n'étant pas en communication fluidique avec ledit passage à trois voies ;
un premier tube (212) raccordé audit premier orifice (204) et en communication fluidique avec celui-ci ;
un deuxième tube (216) raccordé audit deuxième orifice (206) et en communication fluidique avec celui-ci ;
et
un troisième tube (220) raccordé audit troisième orifice (208) et en communication fluidique avec celui-ci, ledit troisième tube (220) étant dimensionné et conformé pour pouvoir être raccordé à une source de vide,
**caractérisé en ce que** ledit premier tube (212) se termine par un premier élément (214) bouchon, ledit deuxième tube (216) se termine par un deuxième élément (218) bouchon, ledit quatrième orifice (210) est dimensionné et conformé pour recevoir l'un parmi ledit premier élément (214) bouchon et ledit deuxième élément (218) bouchon et pour permettre une quantité prédéterminée de fluide de passer par un chemin (A, B) de fuite quand elle est reçue, ledit chemin (A, B) de fuite étant formé dans ledit collecteur (202).

2. Raccord selon la revendication 1, dans lequel le troisième tube (220) se termine par un troisième élément (222) bouchon.

3. Raccord selon la revendication 2, dans lequel chacun desdits premier et deuxième éléments (214, 218) bouchon est adapté pour se raccorder audit quatrième orifice (210) afin de sceller de manière sensiblement étanche lesdits éléments (214, 218) bouchon avec ledit quatrième orifice (210).

4. Raccord selon la revendication 2 dans lequel le troisième élément (222) bouchon est dimensionné et conformé pour pouvoir être raccordé à une source (102) de vide.

5. Raccord selon les revendications 1 ou 4, dans lequel lesdits premier, deuxième et troisième tubes (212, 216, 220) sont raccordés de manière non amovible audit collecteur (202).

6. Raccord selon les revendications 1 ou 4, dans lequel ledit quatrième orifice (210) comprend un joint torique disposé dans celui-ci, ledit joint torique comprenant un passage (302) formé sur celui-ci pour permettre à de l'air d'être aspiré au-delà de celui-ci.

7. Raccord selon les revendications 1 ou 4, dans lequel ledit passage (302) à trois voies est un passage en forme de T ou en forme de Y formé dans ledit collecteur (202).

8. Raccord selon la revendication 1, dans lequel le chemin (A, B) de fuite est formé dans le quatrième orifice (210).

9. Système permettant un tirage du lait en mode simple ou en mode double, comportant :
une source de vide (102) intermittente ;
une première téterelle (108) ;
une deuxième téterelle (110) ; et
un raccord (200) selon l'une des revendications 1 à 8, comprenant un corps de raccord présentant lesdits quatre orifices (204, 206, 208, 210) étant espacés les uns des autres, ledit premier tube (212) s'étendant depuis ledit premier (204) desdits quatre orifices avec l'extrémité (214) dudit premier tube adaptée pour se raccorder à l'autre desdites première et deuxième téterelles (108, 110), ledit deuxième tube (216) s'étendant depuis ledit deuxième (206) desdits quatre orifices avec l'extrémité (218) dudit deuxième tube adaptée pour se raccorder à l'une desdites première et deuxième téterelles (108, 110), ledit troisième tube (220) s'étendant depuis ledit troisième (208) desdits quatre orifices avec l'extrémité (222) dudit troisième tube adaptée pour se raccorder à ladite source de vide (102) intermittente, ledit passage étant formé dans un corps central dudit raccord (200), et ledit passage se raccordant de manière fluidique auxdits premier, deuxième et troisième tubes (212, 216, 220), et ledit quatrième (210) desdits quatre orifices étant adapté pour recevoir une desdites première et deuxième extrémités de tube et servant à sceller de manière sensiblement étanche ladite extrémité de tube reçue et à permettre la fuite d'une quantité prédéterminée de fluide par un chemin (A, B) de fuite, ledit chemin de fuite étant formé dans ledit quatrième orifice (210).

10. Système selon la revendication 9, dans lequel lesdits premier, deuxième et troisième tubes (212, 216, 220) sont raccordés de manière non amovible audit corps de raccord.

11. Système selon la revendication 9, dans lequel ledit passage est un passage en forme de T ou en forme de Y.

12. Système selon la revendication 9, dans lequel ledit quatrième (210) desdits quatre orifices est sensiblement fermé de manière étanche et pourvu d'une quantité prédéterminée de fuite.
